# EUROPEAN PATENT SPECIFICATION

(11) **EP 4 200 588 B1**
(45) Date of publication and mention of the grant of the patent: **14.10.2026**
(21) Application number: 21782790.6
(22) Date of filing: 19.08.2021
(51) Int. Cl.: G01N 1/22

(54) **INDOOR AIR QUALITY MONITOR**
INNENRAUMLUFTQUALITÄTSMONITOR
DISPOSITIF DE SURVEILLANCE DE LA QUALITÉ DE L'AIR INTÉRIEUR

(30) Priority: 24.08.2020 GB 202013185
(43) Date of publication of application: 28.06.2023
(73) Proprietor: Flutterlab Ltd, Chester CH2 3QT (GB)
(72) Inventor: MUNRO, Nicholas, Chester, CH2 3QT (GB); D'AMONE, Gabriele, London SW7 (GB)
(74) Representative: Bartle Read
(86) International application number: PCT/GB2021/052152
(87) International publication number: WO 2022/043664

(56) References cited:
- EP-A1- 3 667 194
- EP-A2- 3 258 241
- WO-A1-2015/009350
- GB-A- 2 539 449
- US-A- 3 833 305

## Description

The present invention relates to monitoring of indoor air quality.

Indoor air quality is a major concern to building managers, businesses and indeed to individuals, given the impact it can have on the health of the occupants of a building and on their productivity. A range of different pollutants is commonly found in the built environment that can impact on human health and wellbeing. A report from the World Health Organisation in 1984 suggested that up to 30% of new and remodelled buildings may be subject to complaints related to poor air quality. Symptoms thought to arise from so-called "sick building syndrome" can include headache; eye, nose and throat irritation; fatigue; dizziness and nausea. It is though that poor IAQ can have a major effect on the productivity of a building's occupants. But it is also associated with long-term health problems including respiratory disease, heart disease and cancer.

Levels of pollution are often higher within buildings than outside them. Sources of pollutants include fuel-burning combustion devices, tobacco products, a range of building materials and furnishing materials, cleaning products, and personal care products. Outdoor air pollution can also reach the indoor environment of course. Pollutants in the air inside buildings can include (a) air-borne particles e.g. from diesel exhausts, dust and smoke; and (b) volatile organic compounds (VOCs), including formaldehyde, emitted by certain solid and liquid materials commonly found in buildings. Another aspect of IAQ is carbon dioxide concentration. In a poorly-ventilated building, the increased concentration of CO₂ in the air exhaled by the building's occupants can raise its concentration appreciably in the ambient air, and excessive atmospheric CO₂ is believed to have a range of negative impacts on human health.

Air humidity can affect the well-being of a building's occupants. Excessively high and low humidity are both undesirable.

Air quality in a building may be affected by transient events whose effects are short-lived, as for example when a chemical spray is released into the atmosphere.

Other aspects of the indoor environment - not directly related to IAQ - that have implications for the well-being of the occupants include ambient noise and temperature.

There is a host of reasons why it is desirable to monitor IAQ. Monitoring may make possible a range of remedial measures to improve air quality such as increasing ventilation, or identifying and removing sources of pollutants. By monitoring the effectiveness of such measures can be determined. Building managers may wish to monitor air quality as a matter of best practice, to ensure a healthy environment.

Self-contained devices for monitoring IAQ are known in the patent literature and are commercially available.

GB2539449, for example, discloses an air quality monitor with a roughly toroidal hollow housing, sensors being carried on a broadly cuboidal body at its centre. The device is configured to sense VOCs, CO₂ and reducing compounds. It has a fan to exhaust air along the housing's approximate axis of symmetry and an air intake in a side wall. The pattern of air flow that is achieved by this arrangement as the air passes over the sensors is not clear from the document, but it may be assumed that it is somewhat complex, and potentially turbulent in various places.

US2013260668A1 describes a system including an air quality "control module", in the context of control of an HVAC (heating, ventilation and air conditioning) system, but this module is configured to compare incoming air from the outside against outgoing stale air to provide relative measurements.

US2017023457A1 concerns a portable ambient air quality monitor in which particulate material is detected by projecting laser light into an air flow and detecting scattering of that light by suspended particulates, the detection being made using a photodiode.

WO2015/009350A1 describes an environmental monitoring device that includes an imaging device. The imaging device has different spatial sensitivity in different regions of the external environment.

EP3258241A2 discloses a particulate matter sensor device in which an enclosure defines a flow inlet and a flow outlet, and a radiation source is provided for emitting radiation into a flow channel to interact with particulate matter in the flow of an aerosol sample guided through it. The radiation is detected by a suitable detector.

An example of a commercially available IAQ monitor is the *Laser Egg* from Kaiterra (kaiterra.com) which is a self-contained puck-shaped device with a display screen on its face. Numerical simulations carried out by one of the present inventors suggest that air flow through the device is far from optimal.

The inventors have identified three areas of deficiency in the prior art IAQ monitors known to them.

The first of these areas concerns management of air flow through the device. The inventors have recognised that existing devices typically suffer from one or more of the following problems:
- airflow through the device is turbulent and/or contains recirculating zones, which cause local variations in the residence time of air (and hence of the air-borne pollutants being sensed). The result can be impairment of accuracy of measurement and/or an increase in the response time of the device. That is, poor management of airflow can impair the ability of the device to react quickly to changes of air quality, with the result that transient effects may be missed altogether;
- inhomogeneous flow. The rate of air flow through the device may vary markedly from one point to another, which is undesirable in terms of accuracy;
- excessive chamber volume. A chamber of large volume - and especially of large cross section with respect to the flow - increases the time it takes for air in the chamber to reach equilibrium with the ambient air, and so increases the response time of the device;
- poor optimisation of flow with respect to the sensors. Certain sensors perform optimally when airflow passes across (parallel to) their upper face. This optimal flow pattern is not achieved in many existing devices.

Since an IAQ monitor is typically a mass-produced item whose cost of manufacture is important to its commercial success, suitable management of airflow needs to be achieved in a manner that is constructionally simple and economical.

A second problem associated with many prior art devices concerns calibration and maintenance. Certain sensors remain accurate for a finite period before they require re-calibration or replacement. A means is needed to facilitate this process in a way that is quick and convenient for the user. Sensors may also be affected by accretion of dust or by contamination.

A third problem concerns the manner in which air quality information is conveyed to users. Certain known devices are configured, for example, to output air quality data digitally through a WiFi (RTM) network to an application running on a mobile device. It is also known to route data through a wide area network to a remote server which collates and analyses data from multiple sources. And IAQ monitors are known which carry electronic displays to provide a range of data. But it is likely that many occupants of a room will pay little attention to an IAQ monitor from day to day, and the inventors have recognised that a very simple and conspicuous signalling system is desirable in this context, to give users reassurance when air quality is good and an immediate and clear signal when it is poor. This needs to be achieved in a manner that is simple in terms of manufacture, and is able to provide an aesthetically attractive effect.

According to the present invention, there is an indoor air quality monitor according to appended claim 1 comprising:
a housing providing an air intake leading via a flow path, which is formed within the housing and which comprises a flow chamber, to an air outlet;
a fan arranged to propel air from the air intake through the flow path to the outlet;
at least one sensor which is for sensing at least one air pollutant, the sensor being exposed to air in the flow chamber; and
an air guide arrangement which is downstream of the air intake in the flow path and which forms a plurality of flow passages through each of which air passes in operation of the indoor air
quality monitor to reach the flow chamber, wherein the sensor is disposed on a circuit board, and the flow chamber is defined by:
   the circuit board;
   a housing panel; and
   a pair of peripheral air guides extending from the circuit board to the housing panel,
the peripheral air guides dividing the circuit board into a first zone which is exposed to the airflow and which contains the sensor and a second zone which is not subject to the airflow and which contains electronics other than the sensor.

By dividing the air flow between a plurality of flow passages upstream of the flow chamber, the present invention makes it possible to exercise control over the pattern of air flow in that chamber, and especially can provide a substantially laminar and homogenous flow pattern. This makes it possible to reduce residence time in the flow chamber and correspondingly to reduce the time needed for the monitor to react to changes in ambient pollutant levels.

In a preferred embodiment, the sensor is mounted on a carrier which is removably mountable within the housing, enabling the carrier to be withdrawn from the housing for replacement, servicing and/or calibration of the sensor.

By mounting the sensor on a removable carrier, the invention provides for the sensor to be conveniently removed from the housing for calibration, servicing and/or replacement.

The interior of the housing may be divided by an arrangement of air guides into (a) a first zone forming an air flow path from the air intake to the air outlet, the first zone including the flow chamber and containing the at least one sensor; and (b) a second zone which is isolated from the flow path, the second zone containing the microprocessor and the interface device.

By dividing the space within the housing into a zone that receives the flow of air and contains the sensor, and a separate zone that is excluded from the air flow, this aspect of the inventions provides various advantages. Potentially sensitive electronic components are protected from contamination by the air flow. Any contamination of the air flow itself by such components is prevented. Effects such as heating due to energy given off by the active electronic components including the microprocessor and the wireless interface, which might otherwise have a bearing on outputs of certain sensors, are prevented.

The indoor air quality monitor may comprise an illuminable signal panel; and at least one electrically driven light source for illuminating the signal panel, wherein the hue and/or other visible quality of the light emitted by the light source is adjustable by the microprocessor,
the microprocessor being further configured to adjust the quality of the light emitted by the light source in dependence on the categorisation of the air quality.

The illuminable signal panel may comprise an edge lit transparent or translucent body of glass or polymer material, and can provide a very simple and effective means of signalling the air quality to occupants of a room.

Specific embodiments of the present invention will now be described, by way of example only, with reference to the accompanying drawings, in which:-
Figure 1 shows an indoor air quality monitor ("IAQ monitor") embodying the present invention viewed from the front and to one side;
Figure 2 shows a housing of the IAQ monitor;
Figure 3 shows a base part of the housing and certain components carried thereupon, these being revealed by omission of a cover part of the housing;
Figure 4 is similar to Figure 3 but includes a circuit board;
Figure 5 includes, on the left-hand side, a view of the underside of the cover portion of the housing, and on the right-hand side a view of the housing and its internal components from which a top panel of the cover has been omitted, to reveal internal features;
Figure 6 shows a view of the housing and its internal components similar to that found in Figure 5, but also includes a set of flow paths generated by computer simulation and representing the routes taken through the housing by air;
Figure 7 illustrates how the housing can be opened for renewal of sensor components, and shows three different states of the housing;
Figure 8 is a plan view of the housing with a top panel of the housing omitted to reveal the circuit board and other internal components;
Figure 9 is a view of the whole IAQ monitor viewed from behind and to one side, along with a peripheral unit on which the IAQ monitor is mounted;
Figure 10 is a graph representing the rate at which internal contaminant concentration equalises with external contaminant concentration in (a) the present IAQ monitor and (b) a prior art device numerically modelled by the present inventor; and
Figure 11 is a graph illustrating the time variation of contaminant concentration in these two devices, in a situation where the ambient concentration of the contaminant varies.

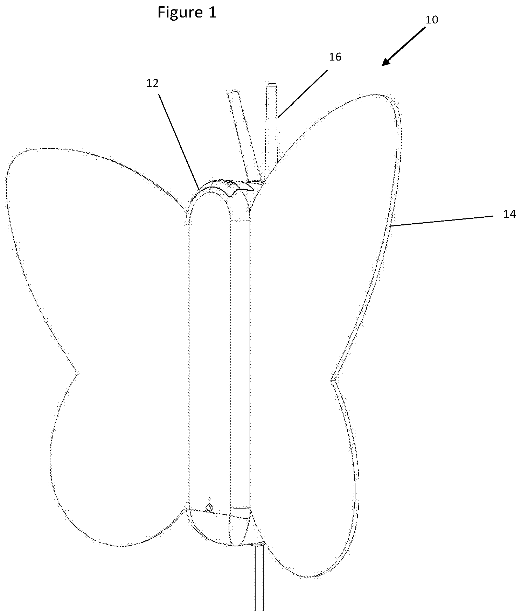

Referring to Figure 1, the present embodiment is an IAQ monitor 10 comprising a housing 12 which in this embodiment carries a pair of illuminable signal panels 14. For the sake of aesthetics, the present embodiment has the general form of a butterfly, where the housing 10, being elongate, represents the creature's body and the signal panels 14 represent its wings. To add to the effect, the housing carries, in the present embodiment, a pair of projections 16 representing antennae. Of course, the outward appearance of the IAQ monitor 10 may be quite different in other embodiments of the invention.

The IAQ monitor 10 carries out repeated and frequent sampling of multiple aspects of the quality of the air in its environment and, in the present embodiment, outputs resultant sensor data through any of (a) an unwired local area network, which includes WiFi (RTM) in the present embodiment as well as the mobile (cellular) telephony network, although other unwired forms of communication may be adopted; (b) a wired local area network; and/or (c) the signal panels 14. The sensor data may subsequently be transmitted through a wide area network which may be the internet.

The IAQ monitor 10 is essentially self-contained, in that it includes all of the functionality needed to sense and report air quality in its environment, although data from multiple IAQ monitors 10 may be collated, as for example where monitoring goes on at multiple locations in a building and data from these locations is transmitted to a server for analysis and reporting.

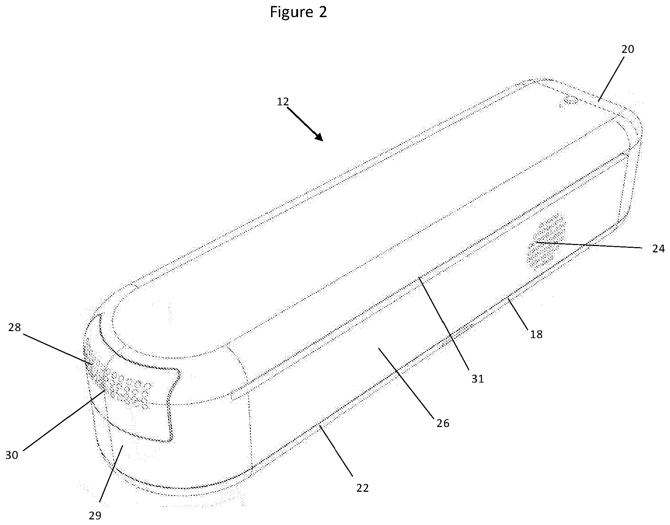

Turning now to Figure 2, the housing 12 comprises a base portion 18 to which a cover portion 20 is releasably coupled, the line of division 22 between these parts being indicated in the drawing. Air is drawn into the housing through a side intake 24 in this embodiment, formed by multiple small apertures in the housing's side wall 26. The lateral direction of the air intake 24 is convenient in terms of packaging in the present embodiment, but in other embodiments the air intake 24 could be differently orientated and in particular it could face along the length of the housing 12. Air is exhausted from the housing 12 through an air outlet 28 formed in this embodiment in an end wall 29 of the housing, and more specifically in a removable closure part 30 of the end wall 29. The air outlet 28 takes the form of multiple small apertures in the closure part 30. Along the side wall 26 (and along its counterpart on the opposite side of the housing, not seen in Figure 2) is an elongate channel 31 which serves to receive an edge of one of the signal panels 14, in order to mount it.

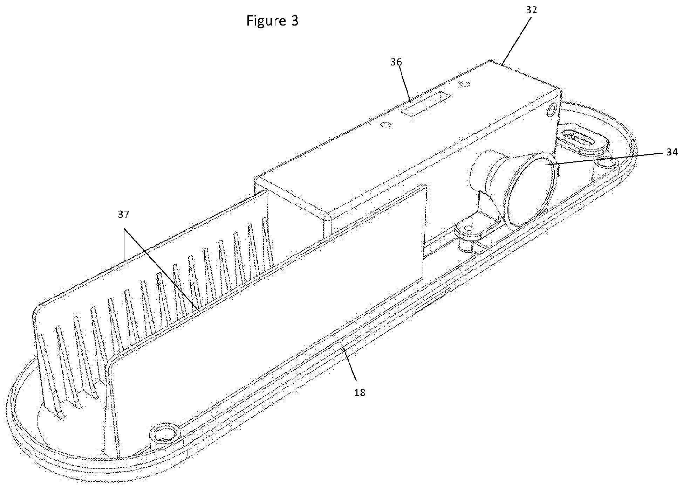

In operation, air is constantly propelled through the housing 12 from the air intake 24 to the air outlet 28 by means of an electrically driven fan. The word "fan" as used herein refers to any mechanism capable of suitably propelling the air but does not require any particular form of mechanism, which may or may not include a rotary device such as an impeller or propeller. The fan is not depicted in Figure 2. In the present embodiment it is incorporated in a commercial particulate sensing unit 32 seen in Figure 3. A frusto-conical duct 34 leads the airflow from the air intake 24 into the particulate sensing unit 32, from which air is exhausted through an upwardly-facing sensor outlet 36.

The particulate sensing unit 32 is of a type known to the skilled person, based on scattering or reflection of emitted light off particles suspended in the air flow, and will not be described in detail herein. Any suitable particulate sensing device may be used in other embodiments of the present invention.

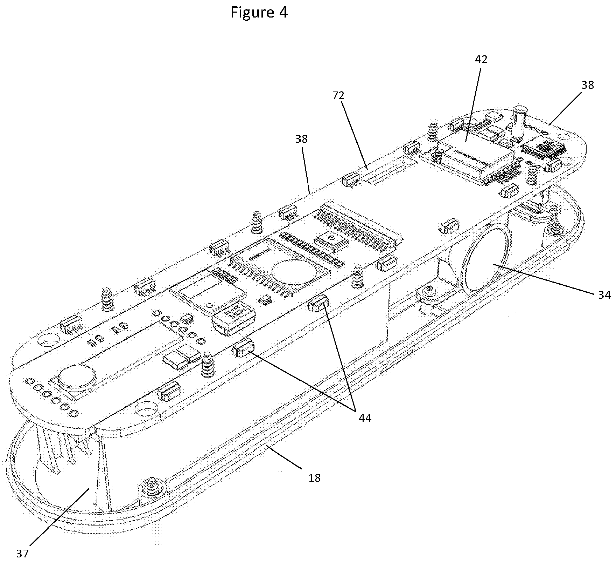

Figure 3 shows a pair of upstands 37 carried by and integrally formed with the housing base portion 18. These serve to support a circuit board 38, which is seen in Figure 4.

The circuit board 38 comprises two spatially separated zones:
i. a first zone 40, which is exposed to the airflow and which contains board-mounted sensors for detection of air quality; and
ii. a second zone 50, 52, 54 which is not subject to the airflow and which contains electronics other than the sensors themselves, among them a microprocessor 42 and associated memory, a WiFi (RTM) chip including its antenna, and also a set of LEDs 44 for illuminating the signal panels 14.

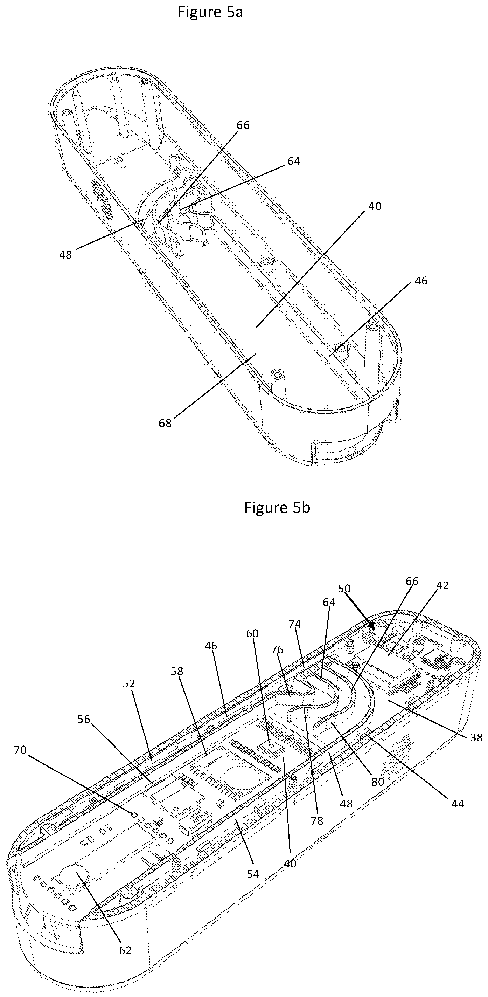

Looking at Figure 5, the extent of the first zone is defined by peripheral air guides 46, 48 between which the airflow is constrained to pass. The first zone 40 lies between the peripheral air guides 46, 48 and occupies a central portion of the circuit board 38, extending along part of its length. The second zone includes an end portion 50 but also two peripheral portions 52, 54 alongside the first zone, forming a rough "U" shape. The peripheral portions 52, 54 contain the LEDs 44.

Within the first zone, and mounted on the circuit board 38, are:
- first and second sensors chemical 56, 58 responsive to volatile organic chemicals ("VOCs"). The two sensors are responsive to different chemicals or classes of chemicals;
- a unit 60 comprising sensors responsive to ambient temperature and relative humidity; and
- a CO₂ sensor 62 responsive to carbon dioxide concentration.

Figure 5b includes the aforementioned peripheral air guides 46, 48 and also a pair of intermediate air guides 64, 66, but this drawing omits a top panel 68 of the cover portion 20 of the housing 12. It should be understood nonetheless that the air guides 46, 48, 64, 66 are carried by, and integral with, the top panel 68, as will be clear from Figure 5a. The lower edges of the air guides 46, 48, 64, 66 rest upon the upper face of the circuit board 38. A flow chamber 70 is thus formed in the region defined between (a) the first zone of the circuit board 38, (b) the peripheral air guides 46, 48 and (c) the top panel 68. It should be noted that this flow chamber is of shallow depth (measured perpendicularly to the circuit board 38), which has beneficial effects. It serves to minimise the flow chamber's cross-sectional area and volume, thus minimising residence time (of air and of the contaminants it carries) in the flow chamber 70. It also helps to ensure that flow over the sensors 56, 58, 60, 62 passes in the desired direction, parallel to the sensors' upper faces, which has been shown to maximise their effectiveness.

Air flow into and through the flow chamber 70 is carefully managed with a view to maintaining laminar (as opposed to turbulent) flow. The pattern of laminar flow created has no local zones of stagnation or recirculation which might increase the residence time of particles and contaminants in such zones. To enter the flow chamber 70, the air exhausted from the particulate sensing unit 32 passes upwardly through an opening 72 in the circuit board (see Figure 4) after which a curved flow passage formed within a duct arrangement 74 (seen in Figure 5b) diverts the flow through approximately ninety degrees (about an axis parallel to the length of the housing 12), to be parallel to the circuit board and transverse to it. The flow is then diverted through approximately 90 degrees (about an axis perpendicular to the circuit board 38) by curved regions of the air guides 46, 48, 64, 66, to pass into the flow chamber 70 moving in a direction parallel to the circuit board 38 and running along the length of the housing 12.

The shape of the air guides 46, 48, 64, 66 has been the subject of an iterative design process, with numerous designs being modelled and the resultant airflow simulated by finite element analysis, in order to arrive at the design depicted herein. It will be noted that the air guides 46, 48, 64, 66 form, in the region where the air enters the flow chamber 70, multiple flow passages - in the present embodiment there are three such passages 76, 78 and 80. Each of these flow passages diverges somewhat along the direction of flow. The central flow passage 78 diverges more than the peripheral passages 76 and 80 and so has a large cross-sectional at its open mouth leading to the flow chamber 70. This is because flow speed of air entering the central flow passage 78 is somewhat higher than the speed of air entering the peripheral passages 76, 80. This is compensated for by the divergence of the central passage 78. The three passages 76, 78, 80 each have a curved form, without any discontinuities to create turbulence.

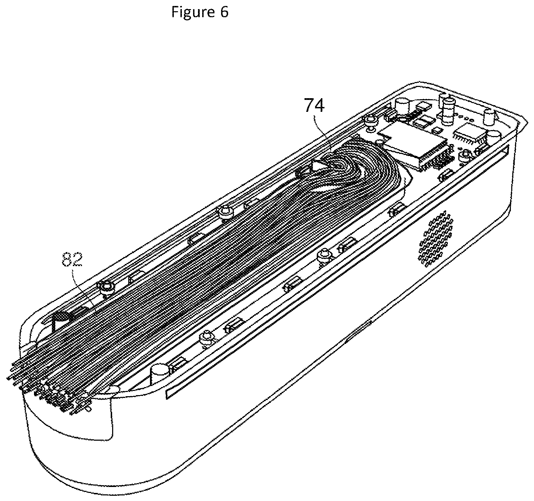

The result, in terms of air flow, is represented in Figure 6, where each of the numerous lines 82 has been created by numerical simulation and represents a path taken by air moving through the passages 76, 78, 80 and through the flow chamber 70. It can be observed that the flow is highly laminar, with no eddies and no observable turbulence. In addition, modelling shows that flow is highly homogeneous, in that the velocity of air flow varies very little across the width, depth and length of the flow chamber 70. If one considers the air flow as a vector field, the flow vectors at any point in the flow chamber are substantially parallel to those at any other point - that is, there is s single direction of flow and the air moves along substantially straight lines.

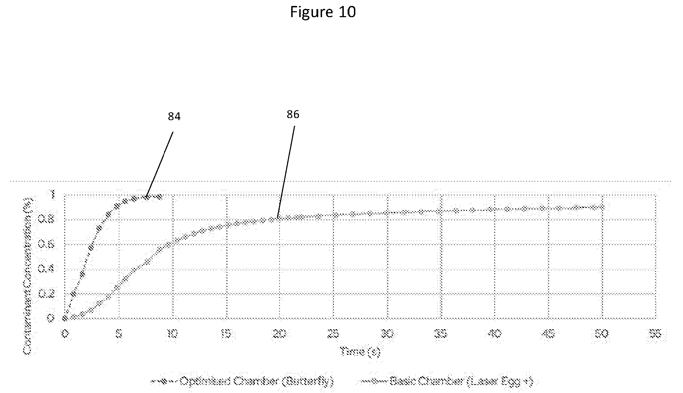

This laminar, homogeneous flow of air through a flow chamber of small flow cross section has the important benefit that that the level of any pollutant inside the flow chamber 70 is able to equalise rapidly with the pollutant level in the ambient air. Figure 10 represents the results of a simulation carried out by one of the inventors using (a) a numerical model of the present IAQ monitor 10, whose results are represented by line 84 and (b) a numerical model of another IAQ monitor, currently among the commercial market leaders, whose results form line 86. The horizontal axis represents time in seconds from introduction of a pollutant into the ambient air. The vertical axis represents the ratio of the pollutant level inside the IAQ monitor to the pollutant level in the ambient air. It can be seen that in the present IAQ monitor 10 the internal pollutant level reaches 90% of the external level within 5 seconds, whereas in the competitor product the same change takes roughly fifty seconds.

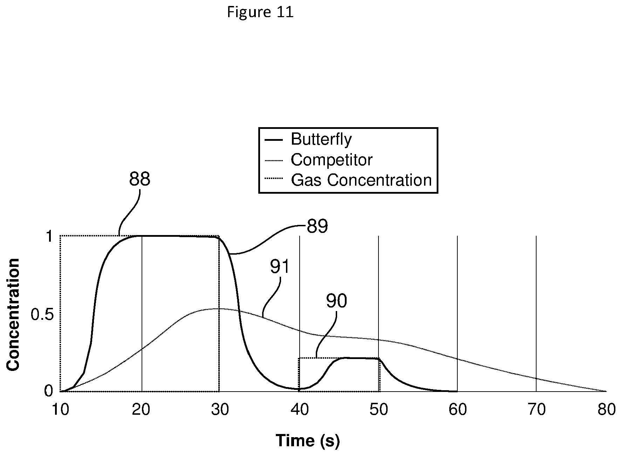

Transient effects on air quality are relevant in assessment of air quality and its effects on health. For example, sprays such as deodorants or cleaning products are released rapidly and quite rapidly dispersed or settled, but may have important effects nonetheless. Hence maximising the rate of the response of the IAQ monitor 10 is important. Figure 11 illustrates the point. Here, boxes 88, 90 represent variations in ambient pollutant level (this example is somewhat simplified in that the pollutant level is modelled as a pair of square pulses.) Lines 89 and 91 represent respectively the responses of the present IAQ monitor 10, and of the aforementioned competitor device. It will be observed that while the present IAQ monitor 10 accurately detects the peak level of the pollutant, the competitor device gives a falsely low reading because it responds so slowly as to fail to reach the true reading. Hence this device potentially gives a falsely reassuring response to such transient events.

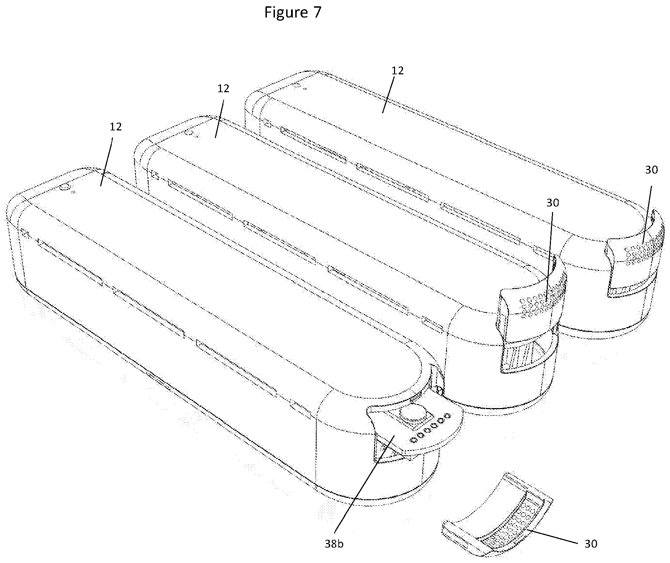

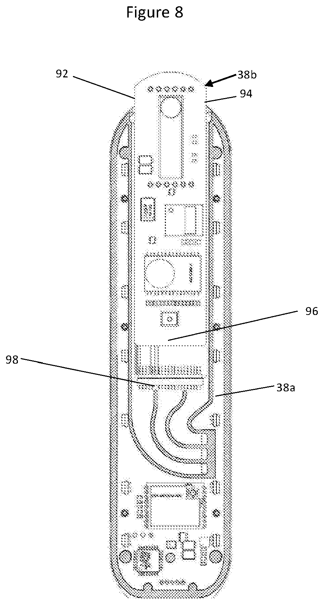

It was noted above that some air quality sensors require periodic renewal, or at least checking and recalibration. This is provided for in the present IAQ monitor 10 by enabling the relevant sensors to be easily removed and replaced. With reference to Figure 8, it must be understood that in the present embodiment the circuit board 38 comprises two separate parts - (a) fixed part 38a and (b) removable part 38b, which forms a sensor module. In the present embodiment the line of division between the fixed and removable parts 38a, 38b corresponds to the division between the aforementioned first and second zones, so that the part of the circuit board corresponding to the first zone and carrying the sensors 56, 58, 60, 62 can be removed for replacement, while the part of the circuit board carrying the remainder of the electronics is fixed in place. Access is gained to the removable circuit board part 38b (hereinafter "the sensor module 38b") by sliding back and removing the closure part 30 of the housing end wall 29, as seen in Figure 7. The sensor module 38b is then able to be slid out through the resultant opening in the housing end wall 29. Figure 8 shows the sensor module 38b partially withdrawn from the housing. Longitudinal edges 92, 94 of the sensor module 38b are received as a sliding fit in corresponding grooves (not seen) in the peripheral air guides 46, 48, guiding the board to its required location, and in-line connectors 96, 98 carried respectively on the sensor module 38b and on the fixed circuit board part 38a are arranged to be brought into engagement when the sensor module 38b is slid fully home, so that this action serves to make the necessary electrical connections.

This modular form of construction allows the sensors to be easily removed. The sensor module 38b can then be replaced or re-calibrated and re-installed, as appropriate. In practice an exchange programme may be implemented, where a sensor unit 38b in need of re-calibration is exchanged for a new or re-calibrated unit.

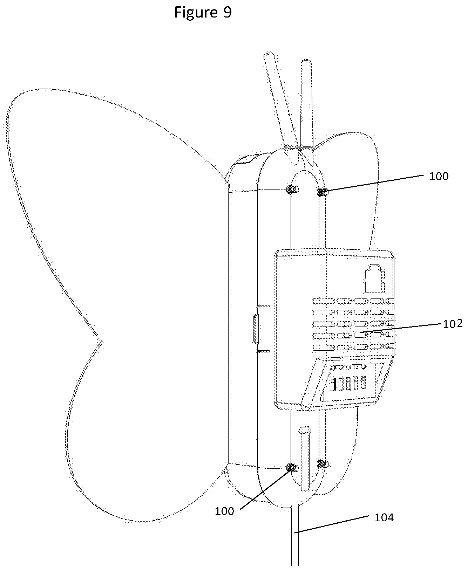

The IAQ monitor 10 will typically be wall-mounted, and Figure 9 shows threaded fasteners 100 which extend through the base portion 18 of the housing for this purpose (once the fasteners have been screwed through the base portion 38 to secure the monitor to the wall, their heads are covered and concealed by fitting of the housing's cover portion 20). However, Figure 9 also shows an optional backpack unit 102 which provides PoE ("Power over Electrical wiring") connectivity to a local area network, which may be used in preference to a wire-free LAN, or in addition to it. Power is here provided from a mains electrical supply through cabling 104, although in other embodiments the IAQ monitor 10 may have an onboard battery, which may provide for continued operation in the event of power outages of limited duration.

Sensor data will typically output through the LAN for processing and provision to users through a suitable dashboard, which may be implemented through an application running on a mobile device and/or on a more substantial computer system, which may be the same system used to manage other aspects of the building's management. The data will also typically be output through a wide area network (which may be the internet) to remote servers at which data from numerous sites is collated. These servers may be run by or for, the supplier of the IAQ monitor 10, for example, that company thus being able to collate and to analyse data from the numerous sites on which its products are installed. This bulk data may provide valuable insights in relation to indoor air quality and to the factors affecting it. It may also have commercial value in itself.

However, the present embodiment also provides a very simple and visually conspicuous indication of air quality through the aforementioned signal panels 14. These panels are illuminated in operation by the LEDs 44 in "edge lit" manner. The effect is widely known: light is directed into an edge of the panel and propagates through it due to partial internal reflection at the panel's faces, so that the panel is seen to glow. In the present embodiment the signal panels 14 comprise acrylic, which is very suitable for this "edge lit" effect. The LEDs each comprise multiple light emitting junctions operating at different wavelengths, so that the colour of the signal panels 14 can be varied under the control of the on-board microprocessor 42. In the present embodiment, this microprocessor is programmed to undertake simple analysis of the sensor output data and to adjust the light provided to the signal panels 14 accordingly. Specifically, the microprocessor may be programmed to select between three states: (a) good air quality, indicated by green light from the display panels 14; (b) moderate air quality, indicated by amber light; and (c) poor air quality, indicated by red light. The display panels give the occupants of a room a clear and immediate signal of the current quality of the local air, and a clear signal to take remedial action if the air quality is poor.

In addition to sensing indoor air quality, the present embodiment is able to sense other aspects of the indoor environment which affect its occupants. Specifically, it has a microphone responsive to ambient noise, and a light sensor. The microphone may be a MEMs type device, although a range of different microphone technologies may be adopted. The light sensor may be a single device, or may comprise multiple sensors responsive to different frequencies. For example independent measurements may be provided of UVa, UVb, infra-red, and of visible light intensity.

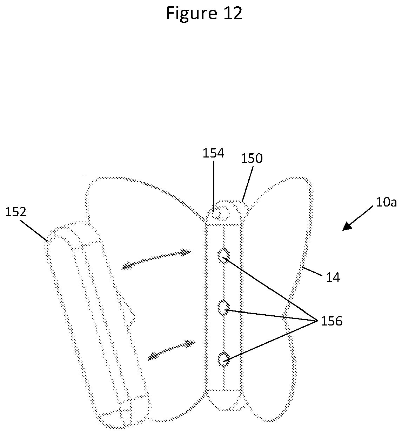

Figure 12 depicts an IAQ monitor 10a which is a further embodiment of the invention and has much in common with the embodiment described above. It differs from the earlier embodiment in that it comprises a base unit 150 and a sensor unit 152, these two units being configured to couple to one another but to be separable. The sensor unit 152 is able to be removed from the base unit 150 and sent for servicing, which may for example include calibration and testing of the sensors. The sensor unit 152 contains the sensors and with regard their mounting and to the management of airflow it may be similar to the earlier embodiment depicted especially in Figures 3 to 7. The base unit 150 is in this example wall-mountable, and it carries the display panels 14, as well as one or more ports for connection to an electrical power supply and, in some embodiments, its wired computer network. In the present embodiment, power to run the sensor unit 152 is transferred from the base unit 150 to the sensor unit 152 through electromagnetic wireless power transmission, a technology already widely adopted in relation to wireless charging of mobile phones. In the illustrated embodiment a power emitter is wholly or partly housed in a stub 154 formed on the base unit 150, and the sensor unit 152 has a complementary recess (not seen in Figure 12) to receive the stub 154 when the two units are coupled. A power receiver is disposed in the sensor unit 152 in the vicinity of the recess. Other embodiments may use conventional electrical connectors, arranged to be mated when the two units 150, 152 are coupled together, for transfer power and/or of data between them. In the illustrated embodiment the base unit 150 carries magnets 156 and the sensor unit 152 carries complementarily arranged magnets which are not seen in the drawings, so that when the two units are brought together, magnetic attraction serves to releasably couple them. Removal of the sensor unit 152 is thus very straightforward - the user simply withdraws it from the base unit 150 with sufficient force to overcome the magnets.

## Claims

1. An indoor air quality monitor (10, 10a) comprising:
a housing (12) providing an air intake (24) leading via a flow path, which is formed within the housing (12) and which comprises a flow chamber (70), to an air outlet (28);
a fan arranged to propel air from the air intake (24) through the flow path to the outlet;
at least one sensor (56, 58, 60, 62) which is for sensing at least one air pollutant, the sensor (56, 58, 60, 62) being exposed to air in the flow chamber (70); and
an air guide arrangement which is downstream of the air intake (24) in the flow path and which forms a plurality of flow passages through each of which air passes in operation of the indoor air quality monitor (10, 10a) to reach the flow chamber (70), wherein the sensor (56, 58, 60, 62) is disposed on a circuit board (38), and the flow chamber (70) is defined by:
the circuit board (38);
a housing panel (68); and
a pair of peripheral air guides (46, 48) extending from the circuit board (38) to the housing panel (68),
the peripheral air guides dividing the circuit board into a first zone which is exposed to the airflow and which contains the sensor and a second zone which is not subject to the airflow and which contains electronics other than the sensor.

2. An indoor air quality monitor (10, 10a) as claimed in claim 1 in which the flow passages each have a cross-sectional area that increases along the direction of air flow.

3. An indoor air quality monitor (10, 10a) as claimed in claim 1 or claim 2 in which there are two peripheral passages (76, 80) disposed on opposite sides of at least one central passage (78), and in which the central passage (78) has at its downstream end a cross-sectional area larger than the individual cross-sectional areas of each of the peripheral passages (76, 80) at their downstream ends.

4. An indoor air quality monitor (10, 10a) as claimed in claim 2 in which the flow passages are each curved to change the direction of the air flow.

5. An indoor air quality monitor (10, 10a) as claimed in claim 4 in which the flow passages change the direction of the air flow by approximately ninety degrees.

6. An indoor air quality monitor (10, 10a) as claimed in any preceding claim in which the circuit board (38) has first and second faces, where in the first face carries the sensor (56, 58, 60, 62) and lies toward the flow chamber (70), and wherein the flow passages are configured to output air into the flow chamber (70) along a direction substantially parallel to the first face.

7. An indoor air quality monitor (10, 10a) as claimed in claim 6 in which the flow path leads through an opening in the circuit board (38), downstream of which the flow passages are each curved to divert the air along a direction substantially parallel to the first face of the circuit board (38).

8. An indoor air quality monitor (10, 10a) as claimed in any preceding claim comprising two or more intermediate air guides (64, 66) each disposed between the peripheral air guides and each extending between the circuit board (38) and the housing panel (68), so that the flow passages are each formed either between a pair of intermediate air guides (64, 66) or between an intermediate air guide (64, 66) and a peripheral air guide,

9. An indoor air quality monitor (10, 10a) as claimed in claim 8 in which the peripheral and the intermediate air guides (64, 66) are each carried by the housing panel (68).

10. An indoor air quality monitor (10, 10a) as claimed in any preceding claim in which the depth of the flow chamber (70) perpendicularly to the circuit board (38) is less than 10mm.

11. An indoor air quality monitor (10, 10a) as claimed in any preceding claim in which the air flow through the flow chamber (70) is substantially laminar.

12. An indoor air quality monitor (10, 10a) as claimed in any preceding claim, comprising at least one VOC sensor.

13. An indoor air quality monitor (10, 10a) as claimed in any preceding claim in which the sensor is mounted on a carrier which is removably mountable within the housing, enabling the carrier to be withdrawn from the housing for replacement, servicing and/or calibration of the sensor, and the carrier (38b) comprises a first electrical connector (96) and the housing (12) contains a second, complementary, electrical connector, the first and second electrical connectors (98) being arranged so that mounting the carrier (38b) in the housing (12) brings them into connection with one another, to form electrical connections to the sensor (56, 58, 60, 62).

14. An indoor air quality monitor (10, 10a) as claimed in any preceding claim, further comprising:
a microprocessor (42) configured to receive and process the sensor's (56, 58, 60, 62) output; and
an illuminable signal panel (14); and
at least one electrically driven light source for illuminating the signal panel (14), wherein the hue and/or other visible quality of the light emitted by the light source is adjustable by the microprocessor (42),
the microprocessor (42) being further configured to adjust the quality of the light emitted by the light source in dependence on the categorisation of the air quality.

15. An indoor air quality monitor (10, 10a) as claimed in claim 14 in which the signal panel (14) comprises a panel of transparent or translucent material which is edge lit by the light source.

## Patentansprüche

1. Innenraumluftqualitätsmonitor (10, 10a), umfassend:
ein Gehäuse (12), das einen Lufteinlass (24) bereitstellt, der über einen Strömungspfad, welcher innerhalb des Gehäuses (12) ausgebildet ist und eine Strömungskammer (70) umfasst, zu einem Luftauslass (28) führt;
einen Lüfter, der so angeordnet ist, dass er Luft vom Lufteinlass (24) durch den Strömungspfad zum Auslass fördert;
mindestens einen Sensor (56, 58, 60, 62), der zum Erfassen mindestens eines Luftschadstoffs dient, wobei der Sensor (56, 58, 60, 62) der Luft in der Strömungskammer (70) ausgesetzt ist; und
eine Luftleitanordnung, die sich im Strömungspfad stromabwärts des Lufteinlasses (24) befindet und eine Vielzahl von Strömungskanälen bildet, durch die jeweils im Betrieb des Innenraumluftqualitätsmonitors (10, 10a) Luft strömt, um die Strömungskammer (70) zu erreichen, wobei der Sensor (56, 58, 60, 62) auf einer Leiterplatte (38) angeordnet ist und die Strömungskammer (70) begrenzt wird durch:
die Leiterplatte (38);
ein Gehäusepanel (68); und
ein Paar peripherer Luftführungen (46, 48), die sich von der Leiterplatte (38) zu dem Gehäusepanel (68) erstrecken,
wobei die peripheren Luftführungen die Leiterplatte in eine erste Zone, die der Luftströmung ausgesetzt ist und den Sensor enthält, und eine zweite Zone unterteilen, die der Luftströmung nicht ausgesetzt ist und andere Elektronik als den Sensor enthält.

2. Innenraumluftqualitätsmonitor (10, 10a) nach Anspruch 1, bei dem die Strömungskanäle jeweils einen Querschnittsbereich aufweisen, der sich entlang der Richtung der Luftströmung vergrößert.

3. Innenraumluftqualitätsmonitor (10, 10a) nach Anspruch 1 oder 2, bei dem zwei periphere Kanäle (76, 80) an gegenüberliegenden Seiten mindestens eines zentralen Kanals (78) angeordnet sind und bei dem der zentrale Kanal (78) an seinem stromabwärtigen Ende einen Querschnittsbereich aufweist, der größer ist als die einzelnen Querschnittsbereiche jedes der peripheren Kanäle (76, 80) an ihren stromabwärtigen Enden.

4. Innenraumluftqualitätsmonitor (10, 10a) nach Anspruch 2, bei dem die Strömungskanäle jeweils gekrümmt sind, um die Richtung der Luftströmung zu ändern.

5. Innenraumluftqualitätsmonitor (10, 10a) nach Anspruch 4, bei dem die Strömungskanäle die Richtung der Luftströmung um etwa neunzig Grad ändern.

6. Innenraumluftqualitätsmonitor (10, 10a) nach einem der vorstehenden Ansprüche, bei dem die Leiterplatte (38) eine erste und eine zweite Seite aufweist, wobei die erste Seite den Sensor (56, 58, 60, 62) trägt und der Strömungskammer (70) zugewandt ist, und wobei die Strömungskanäle konfiguriert sind, um Luft im Wesentlichen parallel zu der ersten Seite in die Strömungskammer (70) abzugeben.

7. Innenraumluftqualitätsmonitor (10, 10a) nach Anspruch 6, bei dem der Strömungspfad durch eine Öffnung in der Leiterplatte (38) führt, stromabwärts derer die Strömungskanäle jeweils gekrümmt sind, um die Luft im Wesentlichen parallel zu der ersten Seite der Leiterplatte (38) abzulenken.

8. Innenraumluftqualitätsmonitor (10, 10a) nach einem der vorstehenden Ansprüche, der zwei oder mehr Zwischenluftführungen (64, 66) umfasst, die jeweils zwischen den peripheren Luftführungen angeordnet sind und sich jeweils zwischen der Leiterplatte (38) und dem Gehäusepanel (68) erstrecken, sodass die Strömungskanäle jeweils entweder zwischen einem Paar von Zwischenluftführungen (64, 66) oder zwischen einer Zwischenluftführung (64, 66) und einer peripheren Luftführung ausgebildet sind.

9. Innenraumluftqualitätsmonitor (10, 10a) nach Anspruch 8, bei dem die peripheren Luftführungen und die Zwischenluftführungen (64, 66) jeweils von dem Gehäusepanel (68) getragen werden.

10. Innenraumluftqualitätsmonitor (10, 10a) nach einem der vorstehenden Ansprüche, bei dem die Tiefe der Strömungskammer (70) senkrecht zu der Leiterplatte (38) weniger als 10 mm beträgt.

11. Innenraumluftqualitätsmonitor (10, 10a) nach einem der vorstehenden Ansprüche, bei dem die Luftströmung durch die Strömungskammer (70) im Wesentlichen laminar ist.

12. Innenraumluftqualitätsmonitor (10, 10a) nach einem der vorstehenden Ansprüche, der mindestens einen VOC-Sensor umfasst.

13. Innenraumluftqualitätsmonitor (10, 10a) nach einem der vorstehenden Ansprüche, bei dem der Sensor auf einem Träger montiert ist, der abnehmbar innerhalb des Gehäuses montierbar ist, was es ermöglicht, den Träger zur Ersetzung, Wartung und/oder Kalibrierung des Sensors aus dem Gehäuse herauszuziehen, und der Träger (38b) einen ersten elektrischen Verbinder (96) umfasst und das Gehäuse (12) einen zweiten, komplementären elektrischen Verbinder enthält, wobei der erste und der zweite elektrische Verbinder (98) so angeordnet sind, dass das Montieren des Trägers (38b) in dem Gehäuse (12) diese miteinander in Verbindung bringt, um elektrische Verbindungen zu dem Sensor (56, 58, 60, 62) herzustellen.

14. Innenraumluftqualitätsmonitor (10, 10a) nach einem der vorstehenden Ansprüche, ferner umfassend:
einen Mikroprozessor (42), der konfiguriert ist, um den Ausgang des Sensors (56, 58, 60, 62) zu empfangen und zu verarbeiten; und
ein beleuchtbares Signalpanel (14); und
mindestens eine elektrisch betriebene Lichtquelle zum Beleuchten des Signalpanels (14), wobei der Farbton und/oder eine andere sichtbare Eigenschaft des von der Lichtquelle emittierten Lichts durch den Mikroprozessor (42) einstellbar ist,
wobei der Mikroprozessor (42) ferner konfiguriert ist, um die Eigenschaft des von der Lichtquelle emittierten Lichts in Abhängigkeit von der Kategorisierung der Luftqualität einzustellen.

15. Innenraumluftqualitätsmonitor (10, 10a) nach Anspruch 14, bei dem das Signalpanel (14) ein Panel aus transparentem oder transluzentem Material umfasst, das von der Lichtquelle kantenbeleuchtet wird.

## Revendications

1. Dispositif de surveillance de la qualité de l'air intérieur (10, 10a) comprenant :
un boîtier (12) fournissant une entrée d'air (24) menant par l'intermédiaire d'un trajet d'écoulement, qui est formé à l'intérieur du boîtier (12) et qui comprend une chambre d'écoulement (70), à une sortie d'air (28) ;
un ventilateur agencé pour propulser de l'air depuis l'entrée d'air (24) à travers le trajet d'écoulement vers la sortie ;
au moins un capteur (56, 58, 60, 62) qui est destiné à détecter au moins un polluant de l'air, le capteur (56, 58, 60, 62) étant exposé à l'air dans la chambre d'écoulement (70) ; et
un agencement de guidage d'air qui est en aval de l'admission d'air (24) dans le trajet d'écoulement et qui forme une pluralité de passages d'écoulement à travers chacun desquels l'air passe lors du fonctionnement du dispositif de surveillance de la qualité de l'air intérieur (10, 10a) pour atteindre la chambre d'écoulement (70), dans lequel le capteur (56, 58, 60, 62) est disposé sur une carte de circuit imprimé (38), et la chambre d'écoulement (70) est définie par :
la carte de circuit imprimé (38) ;
un panneau de boîtier (68) ; et
une paire de guides d'air périphériques (46, 48) s'étendant de la carte de circuit imprimé (38) au panneau de boîtier (68),
les guides d'air périphériques divisant la carte de circuit imprimé en une première zone qui est exposée à l'écoulement d'air et qui contient le capteur et une seconde zone qui n'est pas soumise à l'écoulement d'air et qui contient des composants électroniques autres que le capteur.

2. Dispositif de surveillance de qualité de l'air intérieur (10, 10a) selon la revendication 1 dans lequel les passages d'écoulement présentent chacun une surface de section transversale qui augmente le long de la direction d'écoulement d'air.

3. Dispositif de surveillance de qualité de l'air intérieur (10, 10a) selon la revendication 1 ou la revendication 2, dans lequel se trouvent deux passages périphériques (76, 80) disposés sur des côtés opposés d'au moins un passage central (78), et dans lequel le passage central (78) présente à son extrémité aval une surface de section transversale supérieure aux surfaces de section transversale individuelles de chacun des passages périphériques (76, 80) à leurs extrémités aval.

4. Dispositif de surveillance de qualité de l'air intérieur (10, 10a) selon la revendication 2 dans lequel les passages d'écoulement sont chacun incurvés pour changer la direction de l'écoulement d'air.

5. Dispositif de surveillance de qualité de l'air intérieur (10, 10a) selon la revendication 4 dans lequel les passages d'écoulement changent la direction de l'écoulement d'air d'environ quatre-vingt-dix degrés.

6. Dispositif de surveillance de qualité de l'air intérieur (10, 10a) selon l'une quelconque revendication précédente, dans lequel la carte de circuit imprimé (38) présente des première et seconde faces, dans lequel la première face porte le capteur (56, 58, 60, 62) et est orientée vers la chambre d'écoulement (70), et dans lequel les passages d'écoulement sont conçus pour délivrer de l'air dans la chambre d'écoulement (70) le long d'une direction sensiblement parallèle à la première face.

7. Dispositif de surveillance de qualité de l'air intérieur (10, 10a) selon la revendication 6, dans lequel le trajet d'écoulement mène à travers une ouverture dans la carte de circuit imprimé (38), en aval de laquelle les passages d'écoulement sont chacun incurvés pour dévier l'air le long d'une direction sensiblement parallèle à la première face de la carte de circuit imprimé (38).

8. Dispositif de surveillance de qualité de l'air intérieur (10, 10a) selon l'une quelconque revendication précédente, comprenant deux guides d'air intermédiaires (64, 66) ou plus, chacun disposé entre les guides d'air périphériques et chacun s'étendant entre la carte de circuit imprimé (38) et le panneau de boîtier (68), de sorte que les passages d'écoulement sont chacun formés soit entre une paire de guides d'air intermédiaires (64, 66), soit entre un guide d'air intermédiaire (64, 66) et un guide d'air périphérique,

9. Dispositif de surveillance de qualité de l'air intérieur (10, 10a) selon la revendication 8 dans lequel les guides d'air périphérique et intermédiaire (64, 66) sont chacun portés par le panneau de boîtier (68).

10. Dispositif de surveillance de qualité de l'air intérieur (10, 10a) selon l'une quelconque revendication précédente, dans lequel la profondeur de la chambre d'écoulement (70) perpendiculairement à la carte de circuit imprimé (38) est inférieure à 10 mm.

11. Dispositif de surveillance de qualité de l'air intérieur (10, 10a) selon l'une quelconque revendication précédente, dans lequel l'écoulement d'air à travers la chambre d'écoulement (70) est sensiblement laminaire.

12. Dispositif de surveillance de qualité de l'air intérieur (10, 10a) selon l'une quelconque revendication précédente, comprenant au moins un capteur de COV.

13. Dispositif de surveillance de la qualité de l'air intérieur (10, 10a) selon l'une quelconque revendication précédente, dans lequel le capteur est monté sur un support qui peut être monté de manière amovible à l'intérieur du boîtier, permettant au support d'être retiré du boîtier pour le remplacement, l'entretien et/ou l'étalonnage du capteur, et le support (38b) comprend un premier connecteur électrique (96) et le boîtier (12) contient un second connecteur électrique complémentaire, les premier et second connecteurs électriques (98) étant agencés de sorte que le montage du support (38b) dans le boîtier (12) les amène en connexion l'un avec l'autre, pour former des connexions électriques avec le capteur (56, 58, 60, 62).

14. Dispositif de surveillance de qualité de l'air intérieur (10, 10a) selon l'une quelconque revendication précédente, comprenant en outre :
un microprocesseur (42) configuré pour recevoir et traiter la sortie du capteur (56, 58, 60, 62) ; et
un panneau de signalisation pouvant être éclairé (14) ; et
au moins une source lumineuse commandée électriquement pour éclairer le panneau de signalisation (14), dans lequel la teinte et/ou une autre qualité visible de la lumière émise par la source lumineuse est réglable par le microprocesseur (42),
le microprocesseur (42) étant en outre configuré pour ajuster la qualité de la lumière émise par la source lumineuse en fonction de la catégorisation de la qualité de l'air.

15. Dispositif de surveillance de qualité de l'air intérieur (10, 10a) selon la revendication 14 dans lequel le panneau de signalisation (14) comprend un panneau en matériau transparent ou translucide qui est éclairé par la tranche par la source lumineuse.
